# EUROPEAN PATENT APPLICATION

(11) **EP 3 815 603 A1**
(43) Date of publication of application: **05.05.2021**
(21) Application number: 19210547.6
(22) Date of filing: 21.11.2019
(51) Int. Cl.: A61B 5/0225, A61B 17/135

(54) **METHOD OF DETERMINING HEMOSTATIC PRESSURE IN HEMOSTATIC DEVICE**

(30) Priority: 30.10.2019 KR 20190136827
(71) Applicant: Daesung Maref Co., Ltd, Gunpo-si, Gyeonggi-do 435-862 (KR)
(72) Inventor: LEE, Jai Hwa, 435-862 Gunpo-si, Gyeonggi-do (KR); KIM, Guk Han, 435-862 Gunpo-si, Gyeonggi-do (KR); LEE, Tae Hyeon, 435-862 Gunpo-si, Gyeonggi-do (KR); JUNG, Jae Han, 435-862 Gunpo-si, Gyeonggi-do (KR); KANG, Seung Ho, 435-862 Gunpo-si, Gyeonggi-do (KR)
(74) Representative: Vossius & Partner Patentanwälte Rechtsanwälte mbB

(57) **Abstract**

According to the present disclosure, a method of determining a hemostatic pressure in a tourniquet includes: driving a limb occlusion pressure (LOP) sensor, which measures a pulse signal of a subject, at a first brightness (S1); checking whether the LOP sensor and a main body are connected to each other (S2); providing a first hemostatic pressure from the main body to the tourniquet (S3); checking, on the basis of a result value of the LOP sensor, whether a pulse of the subject is detected (S4); and, when, on the basis of the result value of the LOP sensor, the pulse of the subject is determined as "not detected," providing a hemostatic pressure, which is obtained by increasing the current hemostatic pressure by a safe hemostatic pressure, from the main body to the tourniquet (S7).

To women, infants, or children, for whom, due to having pulse signals weaker than those of adult men, there is great concern about the occurrence of an error in detecting or checking whether hemostasis is achieved according to the related art, the method according to the present disclosure only provides the minimum hemostatic pressure necessary to ensure that hemostasis is achieved, so that skin damage or pain after surgery, which may be caused by compressing a hemostatic site with an excessive pressure, is minimized.

## Description

### CROSS-REFERENCE TO RELATED APPLICATION

This application claims priority to and the benefit of Korean Patent Application No. 10-2019-0136827, filed on Oct. 30, 2019.

### BACKGROUND

### 1. Field of the Invention

The present disclosure relates to a method of determining a hemostatic pressure in a tourniquet, and more particularly, to a method of determining a hemostatic pressure in a tourniquet, the method capable of solving side effects, such as skin damage to a hemostatic site of a subject (patient), which occurs due to compressing the hemostatic site with an unnecessarily high hemostatic pressure, in spite of the current hemostatic pressure already being high enough, to ensure that hemostasis is achieved, and pain or extravasated blood caused by compressing the hemostatic site with an excessive pressure, and capable of providing, to a subject (patient or the like), only the minimum hemostatic pressure necessary to ensure that hemostasis is achieved.

### 2. Discussion of Related Art

Generally, a hemostatic device is a device that allows rapid hemostasis by compressing a body part close to a bleeding site. A pressure-bulb type hemostatic device that allows hemostasis of a bleeding site by placing a pressure bulb nearby the bleeding site, wrapping a band around the skin, and then injecting air into the pressure bulb through an injection hose so that the pressure bulb expands has been mainly used.

The present applicant thought there was a need to solve the side effects associated with the hemostatic device or the tourniquet, such as skin damage to a hemostatic site of a subject, which occurs due to compressing the hemostatic site with an unnecessarily high hemostatic pressure to ensure that hemostasis is achieved, and pain caused by compressing the hemostatic site with an excessive pressure, while ensuring that hemostasis, which is the very purpose of the tourniquet, is achieved.

The related art includes Korean Utility Model Publication No. 20-2016-0000522 (Title of Invention: Portable emergency tourniquet compression, Date of Registration: February 15, 2016) that discloses a technical configuration relating to "a portable emergency tourniquet compression including: a contact band having a predetermined length and a mounting space formed therein; a fixing tube inserted into and mounted in the mounting space of the contact band and having a connection tube, which includes a connection hole through which air is supplied or discharged, mounted in a protruding manner at one side thereof; a fixing member mounted on each of front and rear surfaces of the contact band to fix the contact band and the fixing tube to a compression site or an arterial bleeding site; and an air injector at which an injector connection hole and the connection hole of the connection tube are connected or separated to or from each other and having an air pocket disposed at an upper portion thereof to supply air to the fixing tube.

However, as described above, the related art is not directed to providing a tourniquet capable of solving the side effects such as skin damage to a hemostatic site of a subject, which occurs due to compressing the hemostatic site with an unnecessarily high hemostatic pressure to ensure that hemostasis is achieved, and pain caused by compressing the hemostatic site with an excessive pressure, while ensuring that hemostasis, which is the very purpose of the tourniquet, is achieved.

Further, while muscle mass, the amount, location, or arrangement of fat, and the size and shape of the human body are different for each patient (subject), the related art does not disclose a tourniquet capable of minimizing the side effects for subjects while ensuring that hemostasis is achieved under various conditions.

Meanwhile, the related art also includes Korean Patent Registration No.10-1573852 (Title of Invention: Tourniquet and apparatus including that, Date of Registration: November 26, 2015) that discloses as follows: "The present invention provides a hemostatic tube that is pulled toward an upper portion of a surgical site on a limb to compress blood vessels and control blood circulation, the hemostatic tube including an annular core formed as a solid having flexibility and a ring member wound in a spiral shape around the core and formed in the shape of a coil. The present invention also provides a hemostatic mechanism including: the hemostatic tube; a fabric member provided in a state of being wound around the hemostatic tube and configured to be unfolded along the limb and wrap around the surgical site; and a plurality of pull straps provided in a state of being wound around the hemostatic tube together with the fabric member. According to the present invention, during surgery, hemostasis can be achieved by pulling the hemostatic mechanism, without much force, toward the upper portion of the surgical site, the time taken for achieving hemostasis can be reduced such that the surgical time is shortened, and the formation of wounds and occurrence of pain can be minimized for patients."

However, as described above, the related art is not directed to providing a tourniquet capable of solving the side effects such as skin damage to a hemostatic site of a subject, which occurs due to compressing the hemostatic site with an unnecessarily high hemostatic pressure to ensure that hemostasis is achieved, and pain caused by compressing the hemostatic site with an excessive pressure, while ensuring that hemostasis, which is the very purpose of the tourniquet, is achieved. Further, while muscle mass, the amount, location, or arrangement of fat, and the size and shape of the human body are different for each patient (subject), the related art does not disclose at all a tourniquet capable of minimizing the side effects for subjects while ensuring that hemostasis is achieved under various conditions.

### [Related Art Documents]

### [Patent Documents]

1. Korean Utility Model Publication No. 20-2016-0000522 (Title of Invention: Portable emergency tourniquet compression, Date of Registration: February 15, 2016)
2. Korean Patent Registration No. 10-1573852 (Title of Invention: Tourniquet and apparatus including that, Date of Registration: November 26, 2015)

### SUMMARY OF THE INVENTION

The present disclosure has been devised to solve the above-described problems and is directed to providing a method of determining a hemostatic pressure in a hemostatic device, the method capable of solving side effects, such as skin damage to a hemostatic site of a subject (patient), which occurs due to compressing the hemostatic site with an unnecessarily high hemostatic pressure, in spite of the current hemostatic pressure already being high enough, to ensure that hemostasis is achieved, and pain or extravasated blood caused by compressing the hemostatic site with an excessive pressure, and capable of providing, to a subject (patient or the like), only the minimum hemostatic pressure necessary to ensure that hemostasis is achieved.

The present disclosure is also directed to providing a method of determining a hemostatic pressure in a hemostatic device, the method capable of, for women, infants, or children, for whom, due to having pulse signals weaker than those of adult men, there is concern about the occurrence of an error in detecting or checking whether hemostasis is achieved according to the related art, minimizing skin damage or pain after surgery by providing only the minimum hemostatic pressure necessary to ensure that hemostasis is achieved.

The present disclosure is also directed to providing a method of determining a hemostatic pressure in a hemostatic device, the method capable of, regarding a hemostatic pressure that may vary according to a bodily condition of a patient, the shape and thickness of a compression site, the muscle strength/weakness, and the amount or arrangement of fat even for the same subject or patient, finding out an optimum hemostatic pressure and pressing a compression site with the optimum hemostatic pressure, thereby, while performing a proper hemostatic action, preventing side effects such as pain or a bodily injury caused by compressing a hemostatic site with an excessive pressure.

To achieve the above objectives, according to an embodiment of the present disclosure, a method of determining a hemostatic pressure in a tourniquet includes: driving a limb occlusion pressure (LOP) sensor, which measures a pulse signal of a subject, at a first brightness (S1); checking whether the LOP sensor and a main body are connected to each other (S2); providing a first hemostatic pressure from the main body to the tourniquet (S3); checking, on the basis of a result value of the LOP sensor, whether a pulse of the subject is detected (S4); and, when, on the basis of the result value of the LOP sensor, the pulse of the subject is determined as "not detected," providing a hemostatic pressure, which is obtained by increasing the current hemostatic pressure by a safe hemostatic pressure, from the main body to the tourniquet (S7).

In the checking of, on the basis of the result value of the LOP sensor, whether the pulse of the subject is detected (S4), the pulse may be checked as being present when a pulse signal having a waveform, in which the pulse signal crosses a lower limit, which is an offset value set to be a value other than 0, from bottom to top and then from top to bottom, crosses an upper limit, which is set to be a value higher than the lower limit, from bottom to top and then from top to bottom, and has a peak value, which is a value higher than the upper limit, is detected even one time.

The method may further include, when the pulse is detected from the LOP sensor in Operation S4, providing a hemostatic pressure, which is obtained by slightly increasing the current hemostatic pressure, from the main body to the tourniquet (S5).

After the slightly increased hemostatic pressure is provided to the subject in Operation S5, the method may return to the checking of, on the basis of the result value of the LOP sensor, whether the pulse of the subject is detected (S4).

The method may further include, when the pulse is not detected from the LOP sensor in Operation S4, gradually increasing an optical brightness of the LOP sensor to n brightness levels that are brighter than the first brightness, while gradually increasing the brightness level to the maximum brightness of the LOP sensor, checking, on the basis of the result value of the LOP sensor, whether the pulse of the subject is detected, and, when the pulse of the subject is not detected even when the optical brightness of the LOP sensor is increased, increasing the optical brightness of the LOP sensor slightly more, and, when the pulse is detected from the LOP sensor after the optical brightness of the LOP sensor is increased slightly more, returning to Operation S5 in which the hemostatic pressure, which is obtained by slightly increasing the current hemostatic pressure, is provided from the main body to the tourniquet (S6), wherein n is a natural number 2 or greater.

As a specific example, the method may further include, when n is 2 and the pulse is not detected from the LOP sensor in Operation S4, while gradually increasing an optical brightness of the LOP sensor to a second brightness, a third brightness, and the maximum brightness of the LOP sensor, which are higher than the first brightness, checking, on the basis of the result value of the LOP sensor, whether the pulse of the subject is detected, and, when the pulse of the subject is not detected even when the optical brightness of the LOP sensor is increased, increasing the optical brightness of the LOP sensor slightly more, and, when the pulse is detected from the LOP sensor after the optical brightness of the LOP sensor is increased slightly more, returning to Operation S5 in which the hemostatic pressure, which is obtained by slightly increasing the current hemostatic pressure, is provided from the main body to the tourniquet (S6), wherein the second brightness and the third brightness are values that exist between the first brightness and the maximum brightness of the LOP sensor, and the third brightness is higher than the second brightness.

Operation S7 may include providing the hemostatic pressure, which is obtained by increasing the current hemostatic pressure by the safe hemostatic pressure, from the main body to the tourniquet when the pulse of the subject is not detected even at the maximum brightness of the LOP sensor.

According to another embodiment of the present disclosure, a method of determining a hemostatic pressure in a tourniquet includes: driving a limb occlusion pressure (LOP) sensor, which measures a pulse signal of a subject, at a first brightness (S1); checking whether the LOP sensor and a main body are connected to each other (S2); providing a first hemostatic pressure from the main body to the tourniquet (S3); checking whether a pulse of the subject is detected by the LOP sensor (S4), wherein the pulse is checked as being present when a pulse signal having a waveform, in which the pulse signal crosses a lower limit, which is an offset value set to be a value other than 0, from bottom to top and then from top to bottom, crosses an upper limit, which is set to be a value higher than the lower limit, from bottom to top and then from top to bottom, and has a peak value, which is a value higher than the upper limit, is detected even one time; when the pulse is detected from the LOP sensor, providing a hemostatic pressure, which is obtained by slightly increasing the first hemostatic pressure, from the main body to the tourniquet (S5); when the pulse is not detected from the LOP sensor, while gradually increasing an optical brightness of the LOP sensor to a second brightness, a third brightness, and a fourth brightness, which are higher than the first brightness, checking whether the pulse of the subject is detected from the LOP sensor, and, when the pulse is detected from the LOP sensor after the optical brightness of the LOP sensor is increased slightly more, providing the hemostatic pressure, which is obtained by slightly increasing the first hemostatic pressure, from the main body to the tourniquet (S6), wherein the fourth brightness is the maximum brightness of the LOP sensor, the second brightness and the third brightness are intermediate values that exist between the first brightness and the fourth brightness, and the third brightness is higher than the second brightness; and, when the pulse of the subject is not detected even at the fourth brightness, providing a hemostatic pressure, which is obtained by increasing the current hemostatic pressure by a safe hemostatic pressure, from the main body to the tourniquet (S7).

The method may further include starting all over again from Operation S1 when an event, in which a value measured by a sensor of a hemostatic device or a separate external sensor is changed to a threshold value or higher, occurs while the hemostatic pressure, which is obtained in Operation S7 by increasing the current hemostatic pressure by the safe hemostatic pressure, is maintained.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a schematic diagram of a hemostatic device according to the present disclosure; and
FIG. 2 is a flowchart of a method of determining a hemostatic pressure in the hemostatic device according to the present disclosure.

### DETAILED DESCRIPTION OF EXEMPLARY EMBODIMENTS

Hereinafter, exemplary embodiments according to the present disclosure will be described in detail with reference to the accompanying drawings. Before proceeding, it should be noted that the terminologies and words used on this specification and in the claims are not to be interpreted solely as the general or dictionary meanings, and they should be interpreted as the meanings and the concept which correspond with the technological ideas of the present disclosure based on the principle that the inventor may properly define the concept of the terminologies in order to explain his or her own invention in the best possible way.

Therefore, the configurations described in the embodiments and the drawings of this specification are merely the most preferred types of embodiments and they do not represent the entire technological ideas of the present invention, and thus, it should be understood that there can be a variety of equivalents and alterations, which can replace these embodiments at the time of filing this application.

### (Configuration of hemostatic device)

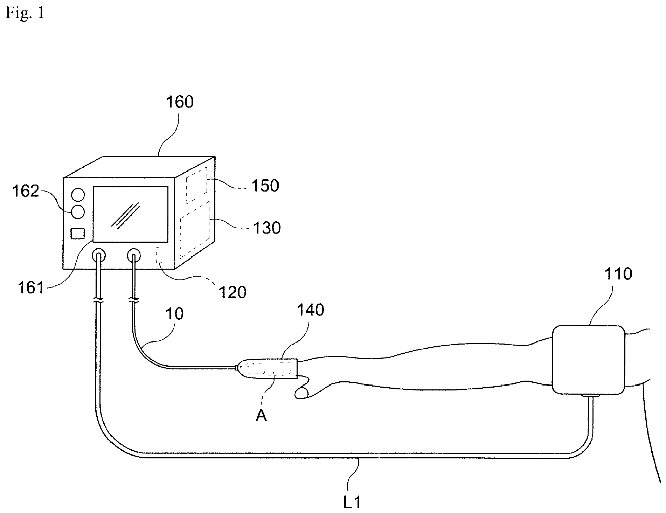

FIG. 1 is a schematic diagram of a hemostatic device according to the present disclosure.

As illustrated in FIG. 1, the hemostatic device according to an exemplary embodiment of the present disclosure includes a cuff 110, which constitutes a tourniquet, a pressure sensor 120, a pressure pump 130, a hemostatic state measurer 140, and a controller 150.

First, the cuff 110 constituting the tourniquet is a compression means that generates a hemostatic pressure required to allow hemostasis of a bleeding site during surgery or first aid treatment. The cuff 110 is mounted around a body part close to a bleeding site and generates a hemostatic pressure by expanding due to air injected thereinto.

Here, the cuff 110 may have an outer shape in the form of a band having a predetermined length to wrap around a body part such as an arm or a leg and may have a fixing means, such as a hook-and-loop fastener or a snap button, provided at an end portion to adjust a size of a circumference of the cuff 110 and fix the adjusted state. Also, a sealed airbag (not illustrated) is disposed along a circumference of an inner portion of the cuff 110. The airbag is connected to the pressure pump 130 through a pneumatic line L1. When air is injected into the airbag, the airbag expands, the circumference of the inner portion of the cuff 110 decreases, and the hemostatic pressure, with which the body part wrapped by the inner portion of the cuff 110 is compressed, increases. When the injected air is recovered, the airbag contracts, the circumference of the inner portion of the cuff 110 increases, and the hemostatic pressure decreases.

The pressure sensor 120 is a sensor which senses the extent to which the cuff 110 compresses a compression site of the body, that is, the hemostatic pressure. The pressure sensor 120 may be mounted on the cuff 110 and directly sense a hemostatic pressure applied to the body or may be disposed on a main body 160 or the pneumatic line L1, which will be described below, and measure a pneumatic pressure supplied to the airbag of the cuff 110 to indirectly sense the hemostatic pressure. A sensed pressure detection value is transmitted to the controller 150 through a separate signal line connected to the controller 150 and used as basic data required to adjust an amount of injected air.

The pressure pump 130 is disposed at an inner portion of the main body 160 to inject air required for the cuff 110 to expand. The pressure pump 130 is connected to the cuff 110 through the pneumatic line L1 and injects air into the cuff 110 by pumping air according to a control signal from the controller 150.

Although not illustrated, a pressure tank in which air discharged from the pressure pump 130 is temporarily stored is provided at the inner portion of the main body 160. The pneumatic line L1 disposed between the pressure tank and the cuff 110 simultaneously serves as, through a single line, an injection tube through which air is supplied toward the cuff 110 and a recovery tube through which the air injected into the cuff 110 is recovered toward the pressure tank. Here, unlike in FIG. 1, the injection tube and the recovery tube may also be formed as two or more separate tubes. The tubes may be used as either an injection tube or a recovery tube according to control of a controller and be controlled to operate in different time slots.

Further, an electronic control valve (not illustrated), which is opened or closed according to a control signal from the controller 150, may be disposed in an injection tube L1 and a recovery tube L1 so that the amount of air injected into the cuff 110, the timing at which the air is injected, the amount of air recovered to the pressure tank, and the timing at which the air is recovered are adjusted.

The hemostatic state measurer 140 is a means for measuring a value which estimates the extent to which blood flows after passing through a compression site of the body that is compressed by the cuff 110, that is, a value which estimates a hemostatic state of a bleeding site. The hemostatic state measurer 140 is mounted on one side of the body part in a direction in which hemostasis is performed by the cuff 110 so as to measure the hemostatic state of the bleeding site. A measured hemostatic state measurement value is transmitted to the controller 150 and used as basic data for adjusting the hemostatic pressure. In the present disclosure, the hemostatic state is measured using an LOP sensor.

The controller 150 is a control means which controls a hemostatic pressure to reach a hemostatic pressure optimal for a patient by using the data obtained by the hemostatic state measurer 140 and the data obtained by the pressure sensor 120.

### (Method of determining hemostatic pressure in hemostatic device)

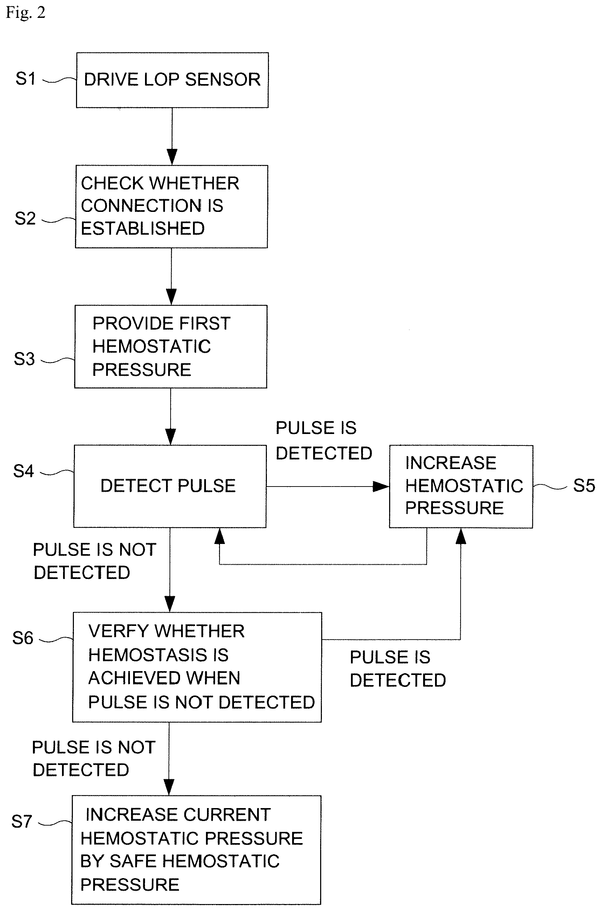

FIG. 2 is a flowchart of a method of determining a hemostatic pressure in the hemostatic device according to the present disclosure.

As illustrated in FIG. 2, the method of determining the hemostatic pressure in the hemostatic device according to the present disclosure includes driving a limb occlusion pressure (LOP) sensor, which measures a pulse signal of a subject, at a first brightness (S1). Here, whether the LOP sensor and a main body are connected to each other is checked (S2). Then, a first hemostatic pressure is provided to a tourniquet (cuff) according to control of a controller of the main body (S3). In this case, the controller checks, on the basis of a result value of the LOP sensor, whether a pulse of the subject (patient) is detected (S4). Then, when, on the basis of the result value of the LOP sensor, the controller determines that the pulse of the subject or patient is not detected, a hemostatic pressure, which is obtained by increasing the current hemostatic pressure by a safe hemostatic pressure, is provided from the main body to the tourniquet (S7).

Here, in the checking of, on the basis of the result value of the LOP sensor, whether the pulse of the subject is detected (S4), the pulse is checked as being present when a pulse signal having a waveform, in which the pulse signal crosses a lower limit, which is an offset value set to be a value other than 0, from bottom to top and then from top to bottom, crosses an upper limit, which is set to be a value higher than the lower limit, from bottom to top and then from top to bottom, and has a peak value, which is a value higher than the upper limit, is detected even one time. Here, the lower limit and the upper limit may be values preset by a designer of the hemostatic device, but the set values may also be changed by an artificial intelligence algorithm in the controller.

The method of determining the hemostatic pressure in the hemostatic device according to the present disclosure further includes, when the pulse is detected from the LOP sensor in Operation S4, providing a hemostatic pressure, which is obtained by slightly increasing the first hemostatic pressure because the first hemostatic pressure is the current hemostatic pressure in an initial stage, from the controller of the main body to the tourniquet or the cuff (S5).

The method of determining the hemostatic pressure in the hemostatic device according to the present disclosure further includes, when the pulse is not detected from the LOP sensor in Operation S4, gradually increasing an optical brightness of the LOP sensor to n brightness levels that are brighter than the first brightness, while gradually increasing the brightness level to the maximum brightness of the LOP sensor, checking, on the basis of the result value of the LOP sensor, whether the pulse of the subject is detected, and, when the pulse of the subject is not detected even when the optical brightness of the LOP sensor is increased, increasing the optical brightness of the LOP sensor slightly more, and, when the pulse is detected from the LOP sensor after the optical brightness of the LOP sensor is increased slightly more, returning to Operation S5 in which the hemostatic pressure, which is obtained by slightly increasing the current hemostatic pressure, is provided from the controller of the main body to the tourniquet or the cuff (S6). Here, n is a natural number 2 or greater.

Specifically, the method may further include, when n is 2 and the pulse is not detected from the LOP sensor in Operation S4 or Operation S5, while gradually increasing an optical brightness of the LOP sensor to a second brightness, a third brightness, and the maximum brightness of the LOP sensor, which are higher than the first brightness, checking, on the basis of the result value of the LOP sensor, whether the pulse of the subject is detected, and, when the pulse of the subject is not detected even when the optical brightness of the LOP sensor is increased, continuing to increase the optical brightness of the LOP sensor slightly more, and, when the pulse is detected from the LOP sensor after the optical brightness of the LOP sensor is increased slightly more, returning to Operation S5 in which the hemostatic pressure, which is obtained by slightly increasing the current hemostatic pressure, is provided from the controller of the main body to the tourniquet or the cuff (S6). Here, the second brightness and the third brightness are values that exist between the first brightness and the maximum brightness of the LOP sensor, and the third brightness is higher than the second brightness.

In this way, by gradually increasing the size of a signal at a light reception sensor side, it is possible to find out the minimum hemostatic pressure necessary to ensure that hemostasis is achieved. To women, infants, or children, for whom, due to having pulse signals weaker than those of adult men, there is great concern about the occurrence of an error in detecting or checking whether hemostasis is achieved according to the related art, only the minimum hemostatic pressure necessary to ensure that hemostasis is achieved may be provided so that skin damage or pain after surgery, which may be caused by compressing a hemostatic site with an excessive pressure, is minimized.

Further, regarding a hemostatic pressure that may vary according to a bodily condition of a patient, the shape and thickness of a compression site, the muscle strength/weakness, and the amount or arrangement of fat even for the same subject or patient, it is possible to find out an optimum hemostatic pressure and press a compression site with the optimum hemostatic pressure, thereby, while performing a proper hemostatic action, preventing side effects such as pain or a bodily injury caused by compressing a hemostatic site with an excessive pressure.

Here, setting of n may vary according to whether the brightness levels of the LOP sensor may be set to be significantly different from one another. That is, the setting of n may depend on whether signal levels in a light reception sensor differ from one another. Generally, the brightness of the LOP sensor may be controlled by current applied thereto. The setting of n may also be affected by performance of the LOP sensor. Even when it is possible to set n as 10, n may be set as 2 as necessary. That is, the brightness may be sequentially set to the first brightness, the second brightness, the third brightness, and the maximum brightness. Further, the setting of n may also be optimally performed using artificial intelligence by the controller of the hemostatic device.

Operation S7 includes providing the hemostatic pressure, which is obtained by increasing the current hemostatic pressure by the safe hemostatic pressure, from the controller of the main body to the tourniquet or the cuff when the pulse of the subject is not detected even at the maximum brightness of the LOP sensor.

The method may further include starting all over again from Operation S1 when an event occurs while the hemostatic pressure, which is obtained in Operation S7 by increasing the current hemostatic pressure by the safe hemostatic pressure, is maintained. Here, the event refers to a case in which a value measured by a sensor of a hemostatic device or a separate external sensor is changed to a threshold value or higher.

The method of determining a hemostatic pressure in a tourniquet according to the present disclosure can solve side effects, such as skin damage to a hemostatic site of a subject (patient), which occurs due to compressing the hemostatic site with an unnecessarily high hemostatic pressure, in spite of the current hemostatic pressure already being high enough, to ensure that hemostasis is achieved, and pain or extravasated blood caused by compressing the hemostatic site with an excessive pressure, and can allow a hemostatic site of a subject (patient or the like) to be compressed with the minimum hemostatic pressure necessary to ensure that hemostasis is achieved.

Particularly, for women, infants, or children, due to having pulse signals weaker than those of adult men, there is great concern about the occurrence of an error in detecting or checking whether hemostasis is achieved according to the related art. For women, infants, or children, to prevent an emergency situation caused by a tourniquet being accidentally released during surgery, a hemostatic site is compressed with an unnecessarily high hemostatic pressure, in spite of the current hemostatic pressure already being high enough, to ensure that hemostasis is achieved. Thus, as described above, skin damage or pain after surgery may occur.

The method of determining a hemostatic pressure in a tourniquet according to the present disclosure can minimize skin damage or pain after surgery for women, infants, or children by allowing a hemostatic site to be compressed with the minimum hemostatic pressure necessary to ensure that hemostasis is achieved.

The method of determining a hemostatic pressure in a tourniquet according to the present disclosure can also, regarding a hemostatic pressure that may vary according to a bodily condition of a patient, the shape and thickness of a compression site, the muscle strength/weakness, and the amount or arrangement of fat even for the same subject or patient, find out an optimum hemostatic pressure and press a compression site with the optimum hemostatic pressure, thereby, while performing a proper hemostatic action, preventing side effects such as pain or a bodily injury caused by compressing a hemostatic site with an excessive pressure.

The present disclosure has been described above using some embodiments and the accompanying drawings, but the present disclosure is not limited thereby. Various modifications or changes may be made by those of ordinary skill in the art to which the present disclosure pertains, within the scope of the technical idea of the present disclosure and the appended claims and their equivalents.

## Claims

1. A method of determining a hemostatic pressure in a hemostatic device, the method comprising:
driving a limb occlusion pressure (LOP) sensor, which measures a pulse signal of a subject, at a first brightness (S1);
checking whether the LOP sensor and a main body are connected to each other (S2);
providing a first hemostatic pressure from the main body to a tourniquet (S3);
checking, on the basis of a result value of the LOP sensor, whether a pulse of the subject is detected (S4); and
when, on the basis of the result value of the LOP sensor, the pulse of the subject is determined as "not detected," providing a hemostatic pressure, which is obtained by increasing a current hemostatic pressure by a safe hemostatic pressure, from the main body to the tourniquet (S7).

2. The method of claim 1, wherein, in the checking of, on the basis of the result value of the LOP sensor, whether the pulse of the subject is detected (S4),
the pulse is checked as being present when a pulse signal having a waveform, in which the pulse signal crosses a lower limit, which is an offset value set to be a value other than 0, from bottom to top and then from top to bottom, crosses an upper limit, which is set to be a value higher than the lower limit, from bottom to top and then from top to bottom, and has a peak value, which is a value higher than the upper limit, is detected even one time.

3. The method of claim 1 or 2, further comprising, when the pulse is detected from the LOP sensor in Operation S4, providing a hemostatic pressure, which is obtained by slightly increasing a current hemostatic pressure, from the main body to the tourniquet (S5).

4. The method of claim 3, wherein, after the slightly increased hemostatic pressure is provided to the subject in Operation S5, the method returns to the checking of, on the basis of the result value of the LOP sensor, whether the pulse of the subject is detected (S4).

5. The method of any one of claims 1 to 4, further comprising, when the pulse is not detected from the LOP sensor in Operation S4, gradually increasing an optical brightness of the LOP sensor to n brightness levels that are brighter than the first brightness, while gradually increasing the brightness level to a maximum brightness of the LOP sensor, checking, on the basis of the result value of the LOP sensor, whether the pulse of the subject is detected, and, when the pulse of the subject is not detected even when the optical brightness of the LOP sensor is increased, increasing the optical brightness of the LOP sensor slightly more, and, when the pulse is detected from the LOP sensor after the optical brightness of the LOP sensor is increased slightly more, returning to Operation S5 in which the hemostatic pressure, which is obtained by slightly increasing the current hemostatic pressure, is provided from the main body to the tourniquet (S6),
wherein n is a natural number 2 or greater.

6. The method of claim 5, further comprising, when n is 2 and the pulse is not detected from the LOP sensor in Operation S4, while gradually increasing an optical brightness of the LOP sensor to a second brightness, a third brightness, and a maximum brightness of the LOP sensor, which are higher than the first brightness, checking, on the basis of the result value of the LOP sensor, whether the pulse of the subject is detected, and, when the pulse of the subject is not detected even when the optical brightness of the LOP sensor is increased, increasing the optical brightness of the LOP sensor slightly more, and, when the pulse is detected from the LOP sensor after the optical brightness of the LOP sensor is increased slightly more, returning to Operation S5 in which the hemostatic pressure, which is obtained by slightly increasing the current hemostatic pressure, is provided from the main body to the tourniquet (S6),
wherein the second brightness and the third brightness are values that exist between the first brightness and the maximum brightness of the LOP sensor, and the third brightness is higher than the second brightness.

7. The method of claim 5 or 6, wherein Operation S7 includes providing the hemostatic pressure, which is obtained by increasing the current hemostatic pressure by a safe hemostatic pressure, from the main body to the tourniquet when the pulse of the subject is not detected even at the maximum brightness of the LOP sensor.

8. The method of claim 7, further comprising starting all over again from Operation S1 when an event, in which a value measured by a sensor of a hemostatic device or a separate external sensor is changed to a threshold value or higher, occurs while the hemostatic pressure, which is obtained in Operation S7 by increasing the current hemostatic pressure by the safe hemostatic pressure, is maintained.
